# EUROPEAN PATENT SPECIFICATION

(11) **EP 3 306 310 B1**
(45) Date of publication and mention of the grant of the patent: **05.08.2020**
(21) Application number: 15894149.2
(22) Date of filing: 01.06.2015
(51) Int. Cl.: G01N 27/62, C12Q 1/37

(54) **METHOD FOR QUANTIFYING MONOCLONAL ANTIBODY**
VERFAHREN ZUR QUANTIFIZIERUNG VON MONOKLONALEN ANTIKÖRPERN
PROCÉDÉ DE QUANTIFICATION D'ANTICORPS MONOCLONAL

(43) Date of publication of application: 11.04.2018
(73) Proprietor: Shimadzu Corporation, Kyoto-shi, Kyoto 604-8511 (JP); National Cancer Center, Tokyo 104-0045 (JP)
(72) Inventor: SHIMADA Takashi, Kyoto-shi Kyoto 604-8511 (JP); IWAMOTO Noriko, Kyoto-shi Kyoto 604-8511 (JP); HAMADA Akinobu, Tokyo 104-0045 (JP)
(74) Representative: Mewburn Ellis LLP
(86) International application number: PCT/JP2015/065806
(87) International publication number: WO 2016/194114

(56) References cited:
- WO-A1-2015/033479
- WO-A1-2015/033479
- JP-A- 2014 510 267
- NORIKO IWAMOTO ET AL: "Selective detection of complementarity-determining regions of monoclonal antibody by limiting protease access to the substrate: nano-surface and molecular-orientation limited proteolysis", ANALYST,, vol. 139, no. 3, 7 February 2014 (2014-02-07), pages 576-580, XP002782006, ISSN: 1364-5528, DOI: 10.1039/C3AN02104A [retrieved on 2013-11-29]
- CHOEI KOBATAKE ET AL.: 'Pharmacological and clinical profile of mogamulizumab (POTELIGEO Injection' FOLIA PHARMACOLOGICA JAPONICA vol. 141, no. 2, 01 February 2013, pages 100 - 105, XP055333552

## Description

### [Technical Field]

The present invention relates to a method for quantifying mogamulizumab using mass spectrometry, and more specifically relates to a method that includes a step of selectively digesting a peptide fragment that includes a specific sequence of mogamulizumab, and a step of detecting a resulting peptide fragment using mass spectrometry, and in which a specific enzyme for selective digestion is used.

### [Technical Background]

Pharmacokinetics, particularly concentration monitoring (therapeutic drug monitoring (TDM) has attracted attention for development and administration of drugs that have less side effects and exhibit high medicinal effects. By concentration monitoring, whether or not an administered drug is in a proper amount and whether or not the drug has reached a lesion site can be confirmed, and effectiveness of the drug can be evaluated and dosage can be appropriately adjusted. Further, it is important that the effectiveness of a molecularly targeted drug, which has become a mainstream in areas such as cancer and autoimmune diseases, can be quickly judged by a doctor himself or herself based on whether or not the drug accumulates at a lesion site and exerts its medicinal effect.

Currently, antibody drugs are attracting attention as molecular target drugs. Antibody drugs specifically bind to cancer and other lesion antigens, target molecules, growth factors, and the like, and thus have attracted attentions as drugs that have less side effects and exhibit high medicinal effects, and clinical studies have been carried out with many antibodies. Antibodies exhibit extremely high molecular specificity due to their nature. On the other hand, even for the same type of cancer, cases are often seen such as a case where antibodies do not exert their medicinal effects merely due to differences in lesion sites, and a case where antibodies are not effective for metastatic lesions. Further, there is also a problem of drug prices, and it is argued that it is important to conduct optimized medical care by proper use of antibody drugs.

Therefore, it is extremely important to measure localization and concentration of an antibody drug and set an optimal dosage. Further, in order to also allow a doctor himself or herself to constantly grasp medicinal effects and establish an aggressive treatment strategy, it is necessary to more easily measure concentration in blood and concentration in a tumor tissue. Further, also for medicinal effect evaluation of an administered drug, development of companion diagnostic agents and the like, there is a great demand for quantification of antibody concentration, and technological innovation is always required.

Conventionally, ELISA (Enzyme-Linked ImmunoSorbent Assay) is known as a most common technique for quantifying proteins such as antibodies. However, there are many problems such as time consuming and being costly. On the other hand, quantitative and structural analysis of proteins using mass spectrometry has been dramatically expanded in its application range, along with developments of mass spectrometry technologies and data analysis servers and software.

A group including the present inventors has found a method that allows an optimal peptide fragment to be obtained for specific detection of a protein such as an antibody using mass spectrometry by selective protease digestion of the protein. This method realizes regioselective protease digestion of a monoclonal antibody by immobilizing both a protein such as an antibody as a substrate and a protease enzyme on solid phases (Patent Document 1 and Non-Patent Document 1).

### [Related Art]

### [Patent Document]

[Patent Document 1] WO 2015/033479.

### [Non-Patent Document]

[Non-Patent Document 1] N. Iwamoto et. al., Selective detection of complementarity determining regions of monoclonal antibody by limiting protease access to the substrate: nano-surface and molecular-orientation limited proteolysis, Analyst, 2014, 139, 576.

### [Summary of the Invention]

### [Problems to Be Solved by the Invention]

In order to easily detect and quantify a protein using a mass spectrometry method, it is efficient to regioselectively cut a protein as a measurement target, produce a peptide fragment specific to the protein, and reduce a production amount of other peptide fragments. Therefore, in a case of an antibody, it is preferable to regioselectively digest an Fab domain, especially a variable region of an Fab domain, in which a specific sequence is included, while suppressing digestion of an Fc domain.

The method described in Patent Document 1 and Non-Patent Document 1 is an innovative method that allows a selective protease digestion of a monoclonal antibody to be performed using a solid phase-solid phase reaction. More specifically, for example, a C-terminal side of an antibody is immobilized onto a Protein G or a Protein A resin having a pore diameter of about 100 nm, and a variable region of the antibody is always oriented to a solution side. Next, a protease is immobilized on surfaces of fine particles having a particle size of about 200 nm. By limiting contact of a protease with the antibody, it is possible to form a reaction field in which a variable region selective antibody decomposition reaction is performed.

However, there is still room for further study in order to actually clinically use the above method, and, in order to provide a simpler quantification method, further improvement such as standardization for individual antibodies is desired.

### [Means for Solving the Problems]

The present inventors have found that, in order to detect an antibody using limiting protease digestion, it is most effective to detect a protease digestion fragment contained in particular in a CDR2 region in a variable region of a monoclonal antibody.

Usually, when a peptide fragment after protease digestion is subjected to mass spectrometry, it is considered to use a protease having less self-digestion and high cleavage sequence selectivity. Therefore, for a person skilled in the art, when a commercially available protease is used, a protease of a mass spectrometry grade or of a sequencing (sequence) grade is used. For example, a native trypsin derived from a living body generates a pseudo trypsin exhibiting chymotrypsin-like activity by self-digestion and thus is known to have low cleavage site specificity. Therefore, a trypsin of a mass spectrometry grade having improved resistance to self-digestion by subjecting a lysine residue of the trypsin to reductive methylation is commercially available.

However, the present inventors have found that, when mogamulizumab as a measurement target has been digested using the above-described so-called high quality protease, quantitative detection may not be possible using an obtained peptide fragment.

The present inventors believe that the above result suggests that there may be interference due to presence of an endogenous antibody to the obtained peptide fragment, and have further studied for a purpose of obtaining a peptide fragment without such interference. As a result, it was surprisingly found that, by using an enzyme with lower specificity than a conventional structural analysis enzyme, the number of cutting sites is increased, peptide fragments of different sequences are generated, and, consequently, optimal peptides for quantitative detection can be obtained, and thus, the present invention is accomplished.

That is, the present invention includes the following aspects.
(1) A method for quantifying a mogamulizumab, the method including: a step of bringing a porous body having pores in which the mogamulizumab is immobilized into contact with fine particles onto which a protease is immobilized, such that selective protease digestion of the mogamulizumab occurs; and a step of detecting a peptide fragment which is obtained from the selective protease digestion and contains an amino acid derived from a CDR2 region of a heavy chain or a light chain of the mogamulizumab, wherein the protease has trypsin activity and chymotrypsin and the fine particles have an average particle size larger than an average pore diameter of the porous body.
(2) The method described in the aspect (1), the protease having trypsin activity and chymotrypsin activity is a trypsin that has not been subjected to a reductive methylation treatment.
(3) The method described in the aspect (1) or (2), the peptide fragment is a peptide having an amino acid sequence expressed in SEQ ID NO: 10.
(4) A kit for quantitative detection of mogamulizumab using liquid chromatography-mass spectrometry (LC-MS), the kit including: a porous body for immobilizing the mogamulizumab; a plurality of fine particles onto which a protease having trypsin activity and chymotrypsin activity is immobilized; a reaction container for bringing the porous body into contact with the fine particles to selectively digest the mogamulizumab; and a buffer solution to be introduced into the reaction container together with the fine particles and the porous body for causing a digestion reaction of the protease to occur.
(5) The kit described in the aspect (4), the protease is a trypsin that has not been subjected to a reductive methylation treatment.
(6) The kit described in (4) or (5), further including an internal standard peptide having an amino acid sequence expressed in SEQ ID NO: 10.
(7) A non-transitory computer readable recording medium that is for use in the method described in any one of the aspects (1)-(3) and in which data for executing mass spectrometry is recorded, wherein the data includes data of a parent ion, a fragment ion, and a voltage applied to electrodes of a mass spectrometer, with respect to a peptide obtained by protease digestion of the mogamulizumab.
(8) A method package for quantitatively detecting mogamulizumab using liquid chromatography-mass spectrometry (LC-MS), the method package including the recording medium described in the aspect (7) and an instruction manual of the recording medium.

### [Effect of the Invention]

Since the method of the present invention uses a limiting protease reaction field, it is possible to selectively decompose and collect a limited region of the mogamulizumab, in particular, the CDR2 region. The CDR2 region is an exposed part closest to a surface of the antibody and thus is preferentially decomposed. Utilizing this fact, detection of mogamulizumab using mass spectrometry can be more efficiently performed. By collecting and quantifying a peptide fragment containing an amino acid of the CDR2 region of mogamulizumab, concentration of mogamulizumab can be directly measured from a biological sample.

Using the method of the present invention, quantification of mogamulizumab using mass spectrometry can be reliably performed. Further, when standard analysis conditions with respect to mogamulizumab are provided, a doctor himself or herself can be involved in analysis of a specimen at a clinical site, and effectiveness of administered mogamulizumab can be promptly evaluated.

### [Brief Description of the Drawings]

Fig. 1 illustrates amino acid sequences (SEQ ID NO: 1 and 2) of a heavy chain and a light chain of mogamulizumab, and sequences of Trypsin Gold cleavage fragments used for quantification and positions of the sequences in the amino acid sequence of the heavy chain or the light chain.
Fig. 2 illustrates MRM chromatograms of Trypsin Gold cleavage fragments (SEQ ID NO: 3 - 9) of mogamulizumab. An upper figure illustrates analysis results of mogamulizumab raw powder, and a lower figure illustrates analysis results of a sample obtained by spiking mogamulizumab into a plasma. A vertical axis indicates a peak intensity and a horizontal axis indicates a retention time. Fragments derived from the same peptide are detected at the same retention time and a difference in production efficiency of a fragment ion is indicated by a peak height.
Fig. 3 illustrates calibration curves prepared using Trypsin Gold cleavage fragments (SEQ ID NO: 3 - 9) of mogamulizumab spiked into a plasma.
Fig. 4 illustrates sequences of Trypsin TPCK-Treated cleavage fragments used in quantification of mogamulizumab, and positions of the sequences in amino acid sequences of a heavy chain and a light chain.
Fig. 5 illustrates MRM chromatograms of Trypsin TPCK-Treated cleavage fragments (SEQ ID NO: 10 - 14) of mogamulizumab. An upper figure illustrates analysis results of mogamulizumab raw powder, and a lower figure illustrates analysis results of a sample obtained by spiking mogamulizumab into a plasma. A vertical axis indicates a peak intensity and a horizontal axis indicates a retention time. Fragments derived from the same peptide are detected at the same retention time and a difference in production efficiency of a fragment ion is indicated by a peak height.
Fig. 6 illustrates calibration curves prepared using Trypsin TPCK-Treated cleavage fragments (SEQ ID NO: 10 - 14) of mogamulizumab spiked into a plasma.
Fig. 7 illustrates structure identification results of mogamulizumab target peptide SYYPDSVK (SEQ ID NO: 10) in precision mass spectrometry.

### [Mode for Carrying Out the Invention]

The present invention relates to a method for quantifying mogamulizumab. The method includes: a step of performing selective protease digestion of mogamulizumab as a measurement target by bringing a porous body in which the mogamulizumab is immobilized in pores into contact with fine particles onto which a specific protease is immobilized, the fine particles having an average particle size larger than an average pore diameter of the porous body; and a step of detecting a peptide fragment that is obtained from the digestion and contains an amino acid derived from a CDR2 region of a heavy chain or a light chain of the mogamulizumab. As the protease, a protease having trypsin activity and chymotrypsin activity is used.

The method of the present invention is intended to reduce population to be analyzed while maintaining specificity of a measurement target. Further, as the protease, not a trypsin that is conventionally regarded as optimal for mass spectrometry, but a protease having trypsin activity and chymotrypsin activity is used. Thereby, a peptide fragment suitable for detection can be obtained.

A region with a particularly high amino acid substitution frequency is referred to as a complementarity determining region (CDR). In the present specification, the term "CDR2 (region)" refers to a region that includes, in addition to a region defined as a CDR2 by a specific consecutive amino acid residue in a primary structure of an antibody, a region that defines specificity of the antibody as a whole in close proximity to the region defined as the CDR2 in a three-dimensional structure of the antibody. It is known that, even in a region commonly referred to as a framework region (FR), there is a difference in sequence depending on an antibody. A sequence of an FR region may also depend on an origin of an antibody protein, a production process, and the like. A person skilled in the art can determine an amino acid sequence containing an amino acid derived from a CDR2 region for efficiently detecting specificity of a target antibody, and predict and select a peptide fragment as a detection target, by confirming multiple alignments of an amino acid sequence between a target antibody and another antibody and cross reactivity between the target antibody and an endogenous antibody.

Further, in the present specification, the term "amino acid sequence containing an amino acid derived from a CDR2 region" means an amino acid sequence containing at least one amino acid derived from a CDR2 region specific to a target antibody, that is, an amino acid sequence containing a portion of the CDR2 region. In an analysis using mass spectrometry, when there is at least one amino acid residue defining specificity, the specificity can be reliably detected. The term "amino acid sequence containing an amino acid derived from a CDR2 region" is not intended to mean an arbitrary amino acid sequence containing "an amino acid derived from a CDR2 region", but is intended to mean that the amino acid sequence as a whole forms a continuous sequence of a portion of an amino acid sequence of a target antibody.

In the following, the method of the present invention is described in detail.

### [Mass Spectrometry]

In order to detect mogamulizumab using mass spectrometry, it is necessary to first extract the mogamulizumab from a biological sample and to dissolve the mogamulizumab in an appropriate solvent. Further, since a molecule of mogamulizumab is large for analysis, the mogamulizumab is decomposed into peptides by protease, and thereafter, mass spectrometry is performed after the peptides are separated using liquid chromatography. A molecular weight of a peptide suitable for analysis is about 1000 - 3000 Da.

When a mogamulizumab molecule is decomposed by protease, peptide fragments in a number far exceeding 200 are generated. Further, when mogamulizumab contained in a biological sample is a measurement target, a huge sample set is involved. In order to analyze and quantify only a target specific sequence from the above huge and complex sample, liquid chromatography having high resolution and good reproducibility is necessary before a mass spectrometry step.

### [Antibody]

A measurement target in the method of the present invention is a monoclonal antibody. A heavy chain and a light chain of a monoclonal antibody are each formed of a constant region and a variable region. The constant regions each have an amino acid sequence that is common to most of antibodies derived from the same species. On the other hand, in each of the variable regions, there are three complementarity determining regions (CDRs) (CDR1, CDR2, CDR3). A three-dimensional structure defined by these regions is involved in specific binding with an antigen, and thereby, an antibody-antigen complex is formed.

As a result of a three-dimensional structure analysis of an antibody, it is confirmed that the CDR regions involved in specific binding with an antigen are positioned substantially outside of an antibody molecule. Among the CDR regions, the CDR2 region is positioned on an outermost side (Fig. 8), and is optimal as a target of the limiting protease digestion of the present invention. For an antibody that is clinically used, an entire amino acid sequence thereof and sequences of the CDRs are disclosed. Further, a person skilled in the art can identify the CDR regions based on amino acid sequence information of the antibody.

A monoclonal antibody that can be a measurement target in the method of the present invention is mogamulizumab. A monoclonal antibody has a molecular diameter of about 14.5 nm.

According to the method of the present invention, especially a CDR2 region of mogamulizumab can be regioselectively protease-digested, and identification and quantitative detection of mogamulizumab can be performed by detecting a resulting peptide fragment of the CDR2 region using mass spectrometry.

### [Porous Body]

A porous body used in the method of the present invention is not particularly limited in material as long as the porous body has a large number of pores, and activated carbon, a porous membrane, porous resin beads, and metal particles can be used. Among these, those capable of site-specifically binding an antibody are preferable.

The pores are not limited in shape. Further, as in a case of a porous membrane, a porous body having pores formed penetrating the porous body can also be used. A size of a pore of a porous body is not particularly limited, and is preferably determined by considering a molecular diameter of an antibody and the like such that, when an antibody is immobilized, a part to be selectively digested is positioned near a surface layer of a pore. An average pore diameter (D2) of a porous body is appropriately set, for example, in a range of about 10 nm - 200 nm and in a range smaller than an average particle size (D1) of fine particles. For example, the average pore diameter (D2) of a porous body is preferably about 20 nm - 200 nm, more preferably in ranges of 30 nm - 150 nm, 40 nm - 120 nm, and 50 nm - 100 nm, and is particularly preferable about 100 nm.

In the present invention, a porous body in which a linker molecule that site-specifically interacts with an antibody is immobilized in a pore of the porous body is preferably used. As a linker molecule, Protein A or Protein G that site-specifically binds with an Fc domain of an antibody is preferably used. By using a porous body in which these linker molecules are immobilized in pores, an Fc domain of an antibody is immobilized in a pore, and an Fab domain is positioned near a surface layer of the pore. In this way, by controlling orientation of an antibody in a pore, regioselective digestion of an Fab domain, particularly, a CDR2, by a protease becomes possible.

A size of a linker molecule is selected such that a selective cleavage site of an antibody is positioned near a surface layer of a pore. A molecular size in a state in which a linker molecule and an antibody are bound to each other is preferably about 0.5 times - 1.5 times, more preferably about 0.6 times - 1.2 times, even more preferably about 0.7 times - 1.1 times, and particularly preferably about 0.8 times - 1 times a pore diameter of a porous body. When a linker molecule is not fixed to a porous body and an antibody is directly bound in pores, it is preferable that a molecular diameter of the antibody and a pore diameter of the porous body satisfy the above relation.

A porous body that can be suitably used in the present invention is not particularly limited. For example, Protein G Ultralink resin (manufactured by Pierce Corporation) and Toyopearl (manufactured by (TOSO Co. Ltd.) can be used. For example, in the case of the Protein G Ultralink resin, it is known that 95% of Protein G bound to resin is in pores.

### [Immobilization of Antibody in Porous Body]

A method for immobilizing mogamulizumab in a pore of a porous body is not particularly limited. An appropriate method can be adopted according to characteristics of the mogamulizumab, the porous body or a linker molecule. For example, when mogamulizumab is immobilized in a porous body in which a protein A or a protein G is immobilized in a pore, by mixing a suspension of the porous body with a solution containing the mogamulizumab, the mogamulizumab can be easily immobilized in a pore.

A quantitative ratio of a porous body to mogamulizumab can be appropriately set according to a purpose. For example, when a quantitative analysis of mogamulizumab is performed, it is desirable that almost the entire mogamulizumab in a sample be immobilized in the porous body. Therefore, it is preferable that a quantitative ratio be set such that an amount of the porous body becomes excessive with respect to an estimated content of the mogamulizumab in the sample.

### [Fine Particles]

In the method of the present invention, fine particles are used for a purpose of immobilizing a protease on surfaces of the fine particles and controlling access of the protease to mogamulizumab immobilized in pores of a porous body. Therefore, the fine particles have a larger average particle size (D1) than the average pore diameter (D2) of the porous body so as not to enter deep into the pores of the porous body.

The fine particles are not particularly limited in shape. However, from a point of view of homogenization of access of the protease to the pores of the porous body, spherical fine particles are preferred. Further, it is preferable that the fine particles have a uniform particle size.

The average particle size (D1) of the fine particles is preferably in a range of 50 nm - 500 nm, and is more preferably 1.2 or more times, even more preferably 1.5 or more times, and particularly preferably 1.8 or more times, for example, about 2 times the average pore size (D2) of the porous body. When the average pore diameter of the porous body is about 30 - 150 nm, the average particle size (D1) of the fine particles is preferably 100 nm or more, and more preferably 150 nm or more. When the average pore diameter of the porous body is about 50 nm - 100 nm, the average particle size of the fine particles is preferably 120 nm or more, more preferably 150 nm or more, and particularly preferably 170 nm or more. From a point of view of improving digestion efficiency by the protease, an upper limit of the average particle size (D1) of the fine particles is preferably 500 nm or less, and more preferably 300 nm or less.

A material of the fine particles is not particularly limited as long as the above protease can be immobilized on surfaces of the fine particles. A metal or a resin can be appropriately used as the material of the fine particles. Further, a metal coated with a resin, or a resin coated with a metal, can also be used.

As a kind of the fine particles, magnetic fine particles that can be dispersed or suspended in an aqueous medium and can be easily recovered from the dispersion or suspension by application of a magnetic field are preferable. Further, from a point of view that aggregation is less likely to occur, magnetic fine particles coated with an organic polymer are more preferable. Examples of base materials of magnetic fine particles include ferromagnetic alloys such as iron oxide (magnetite (Fe₃O₄), maghemite (γ-Fe₂O₃)), and ferrite (Fe/M)₃O₄. In the ferrite (Fe/M)₃O₄, M means a metal ion that can be used together with an iron ion to form a magnetic metal oxide, and typically, Co²⁺, Ni²⁺, Mn²⁺, Mg²⁺, Cu²⁺ and Ni²⁺ are used. Further, examples of the organic polymer coating the magnetic fine particles include polyglycidyl methacrylate (poly GMA), a copolymer of GMA and styrene, polymethyl methacrylate (PMMA) and polymethyl acrylate) (PMA). Specific examples of magnetic nanobeads coated with an organic polymer include FG beads, SG beads, Adembeads and nanomag. As a commercially available product, for example, FG beads (polymer magnetic fine particles having a particle size of about 200 nm obtained by coating ferrite particles with polyglycidyl methacrylate (poly GMA)) manufactured by Tamagawa Seiki Co., Ltd. is suitably used.

In order to suppress adsorption of a nonspecific protein and to selectively immobilize a protease, it is preferable that the fine particles be modified with spacer molecules capable of binding to the protease. By immobilizing a protease via spacer molecules, desorption of the protease from surfaces of the fine particles is suppressed, and regioselectivity of protease digestion is improved. Further, by adjusting a molecular size of a spacer, a protease can be caused to selectively access a desired position of an antibody, and regioselectivity can be improved.

A spacer preferably can bind to protease and does not inactivate a protease. From a point of view of controlling an access range of a protease immobilized on surfaces of fine particles, a spacer preferably has a small molecular diameter. The molecular diameter of the spacer is preferably 5 nm or less, more preferably 3 nm or less, and even more preferably 2 nm or less. Further, a molecular weight of the spacer is preferably 2000 or less, more preferably 1500 or less, and even more preferably 1000 or less.

A spacer molecule having the above molecular diameter and capable of immobilizing a protease is preferably a non-protein, and is preferably a molecule having a functional group at a terminal, examples of the functional group including an amino group, a carboxyl group, an ester group, an epoxy group, a tosyl group, a hydroxyl group, a thiol group, an aldehyde group, a maleimide group, a succinimide group, an azide group, a biotin, an avidin, and a chelate. For example, for immobilization of a trypsin, a spacer molecule having an activated ester group is preferred. Further, of a spacer molecule, as a spacer arm portion other the functional group, a hydrophilic molecule can be used, examples of the hydrophilic molecule including polyethylene glycol and its derivatives, polypropylene glycol and its derivatives, polyacrylamide and its derivatives, polyethyleneimine and its derivatives, poly (ethylene oxide) and its derivatives, and poly (ethylene terephthalic acid) and its derivatives.

Fine particles that are surface-modified with such spacer molecules are also commercially available, and those commercially available fine particles can be used. For example, fine particles modified with a spacer molecule having an ester group (active ester group) activated with N-hydroxysuccinimide is commercially available under a trade name "FG beads NHS" (Tamagawa Seiki Co., Ltd.). The FG beads NHS has a particle size of about 200 nm ± 20 nm, and is very homogeneous as fine particles.

### [Protease]

In the method of the present invention, as a protease to be immobilized on fine particles, a protease having trypsin activity and chymotrypsin activity is used.

A trypsin has high substrate specificity and has Lys or Arg at a C terminal of a peptide after cleavage and thus can homogenize a charge amount and charge localization of a peptide, and is suitable for preparation of a sample for mass spectrometry. Further, a trypsin has a small molecular diameter (about 3.8 nm) and an active site exists inside a molecule. Therefore, a region where the active site can access an antibody is restricted, and regioselectivity of protease digestion can be improved.

The present inventors have found that, by using a protease having trypsin activity and chymotrypsin activity, not a trypsin of a mass spectrometry grade that is usually used, peptide fragments optimal for quantitative detection of mogamulizumab can be obtained.

As a protease used in the method of the present invention, for example, a native trypsin can be used that is derived from a living body and is known to exhibit chymotrypsin-like activity by self-digestion. A trypsin having improved resistance to self-digestion by subjecting a lysine residue of the trypsin to reductive methylation is commercially available as a trypsin of a mass spectrometry grade. However, a trypsin used in the present invention is preferably a trypsin for which such a reductive dimethylation treatment of a lysine residue is not performed.

For example, Trypsin Gold (manufactured by Promega Corporation) is a recombinant trypsin, and has been subjected to a reductive methylation reaction after a TPCK treatment. On the other hand, Trypsin TPCK-Treated (manufactured by Sigma Corporation) has reduced chymotrypsin activity by subjecting a trypsin purified from a bovine to a TPCK treatment. However, the Trypsin TPCK-Treated is a protease that has not been subjected to a reductive dimethylation reaction.

By using a protease having trypsin activity and chymotrypsin activity, the method of the present invention allows mogamulizumab, which cannot be quantitatively detected when a trypsin that does not have chymotrypsin activity (for example, a trypsin of a mass spectrometry grade) is used, to be quantitatively detected using a peptide fragment that cannot be obtained by digestion of a trypsin of a mass spectrometry grade.

As "a protease having trypsin activity and chymotrypsin activity," it is assumed that a trypsin exhibiting chymotrypsin-like activity as described above is used. However, as another embodiment, it is also possible to use a mixture of a trypsin and a chymotrypsin. In this case, as the trypsin, for example, the Trypsin Gold (manufactured by Promega Corporation) can be used; and, as the chymotrypsin, for example, the Trypsin TPCK-Treated (manufactured by Sigma Corporation) can be used. A ratio of the trypsin to the chymotrypsin is preferably used at a ratio of 9:1 - 1:1 in protease activity (U).

Examples of a protease that can be particularly preferably used in the method of the present invention include Trypsin TPCK-Treated (manufactured by Sigma Corporation), and the like.

### [Immobilization of Protease onto Fine Particles]

A method for immobilizing a protease on surfaces of fine particles is not particularly limited. An appropriate method can be adopted according to characteristics of the protease and the fine particles (or spacer molecules modifying the surfaces of the fine particles). For example, when the protease is immobilized on spacer-modified surfaces of the fine particles, by mixing a suspension of the fine particles and a solution containing the protease, the protease can be immobilized on the surfaces of the fine particles. A method of amine coupling of the fine particles and the protease via functional groups of the spacer molecules is preferable. For example, a carboxyl group modifying surfaces of fine particles can be esterified with N-hydroxysuccinimide (NHS) to form an activated ester group to which an amino group of a protease can be bound. This coupling reaction can be performed in the presence of carbodiimide as a condensing agent, examples of the carbodiimide including 1-ethyl-3-(3-dimethylaminopropyl) carbodiimide (EDAC), N,N'-dicyclohexylcarbodiimide (DCC) and bis(2,6-diisopropylphenyl) carbodiimide (DIPC). Further, an amino group of a protease may be bound to an amino group modifying surfaces of fine particles using a cross-linking agent such as glutaraldehyde, bifunctional succinimide, bis(sulfosuccinimidyl) suberate (BS3), sulfonyl chloride, maleimide, and pyridyl disulfide.

The coupling method of the fine particles and the protease via the functional groups of the spacer molecules can be performed by a simple operation of adding a protease solution to a suspension of the fine particles and mixing and stirring the mixture under certain conditions.

After the protease is immobilized on the surfaces of the fine particles, it is preferable to inactivate an active portion that is not bound to the protease on the surfaces of the fine particles.

### [Protease Digestion]

By bringing a porous body in which mogamulizumab is immobilized and fine particles on surfaces of which a protease is immobilized into contact with each other, the mogamulizumab is protease-digested and peptide fragments are produced. The contact preferably occurs in a liquid. Here, the term "liquid" means that a substrate (solid phase) and an enzyme (solid phase) are in contact with each other in a liquid phase, and is intended to mean an aqueous medium suitable for a protease digestion reaction.

Conditions of protease digestion in the present invention are not particularly limited, and conditions similar to general protease digestion can be suitably adopted. For example, it is preferable to incubate usually at a temperature of about 37 °C for about 4 hour - 20 hours in a buffer solution adjusted to a vicinity of an optimum pH of the protease.

A quantitative mixing ratio of the porous body on which the mogamulizumab is immobilized to the fine particles on the surfaces of which the protease is immobilized is not particularly limited, and may be set so as to have an amount of the protease corresponding to an amount of the mogamulizumab. In the present invention, by a combination of the porous body and the fine particles, access between the mogamulizumab and the protease is physically restricted. Therefore, as compared to general protease digestion, it is preferable to increase the amount of the protease. For example, antibody:protease is preferably about 30:1 - 3:1, more preferably about 15:1 - 4:1, and even more preferably about 10:1 - 5:1.

In the method of the present invention, protease digestion is performed in a state in which the mogamulizumab is immobilized on the porous body. Since the peptide fragments produced by the protease digestion exist in the liquid phase, target peptide fragments can be regioselectively obtained without performing an antibody elution or denaturation operation. According to the method of the present invention, it is possible to regioselectively collect a peptide fragment with a simple operation as compared to a conventional method.

Next, a target peptide fragment obtained by protease digestion is detected. For the detection, it is preferable to remove the porous body and the fine particles. This can be achieved by subjecting a sample after the protease digestion to filtration, centrifugal separation, magnetic separation or dialysis. For example, by filtration using a filtration membrane made of polyvinylidene fluoride (PVDF) (low-binding hydrophilic PVDF having a hole diameter of 0.2 µm manufactured by Millipore Corporation), the porous body and the fine particles can be easily removed.

### [Liquid Chromatography-Mass Spectrometry (LC-MS)]

By analyzing a sample containing the above-obtained peptide fragment using LC-MS, identification and quantification of the mogamulizumab can be performed.

For purposes such as more reliably separating the peptide fragment and improving analysis accuracy, a sample before being subjected to mass spectrometry is subjected to separation and concentration using liquid chromatography (LC). When separation of a sample is performed using LC, an eluate from LC may be directly ionized and subjected to mass spectrometry. Analysis can also be performed using LC/MS/MS or LC/MSn combining LC with tandem mass spectrometry. Further, the eluate from the LC may be collected once and then subjected to mass spectrometry. An LC column is not particularly limited, and a hydrophobic column such as C30, C18, C8, and C4 generally used in peptide analysis, and a carrier for hydrophilic affinity chromatography, can be appropriately selected and used.

Mass spectrometry can determine an amino acid sequence and thus can determine whether or not a peptide fragment is a peptide fragment derived from a specific protein such as mogamulizumab. Further, based on a peak intensity, concentration of peptide fragments in a sample can be determined. In performing analysis, a sample may be used for the analysis after being subjected to treatments such as desalting, solubilization, extraction, concentration, and drying when necessary.

An ionization method in mass spectrometry is not particularly limited, and an electron ionization (EI) method, a chemical ionization (CI) method, a field desorption (FD) method, a fast atom collision (FAB) method, a matrix assisted laser desorption ionization (MALDI) method, and an electrospray ionization (ESI) method can be adopted. A method for analyzing an ionized sample is also not particularly limited, and a method of a magnetic field deflection type, a quadrupole (Q) type, an ion trap (IT) type, a time of flight (TOF) type or a Fourier transform ion cyclotron resonance (FT-ICR) type can be appropriately determined according to the ionization method. Further, MS/MS analysis or multistage mass spectrometry of MS3 or higher can also be performed using, for example, a triple quadrupole mass spectrometer.

A mass spectrometer that is suitable for implementing the method of the present invention is not particularly limited. Examples of the mass spectrometer include liquid chromatography-triple quadrupole mass spectrometers such as LCMS-8030, LCMS-8040, and LCMS-8050 (all manufactured by Shimadzu Corporation), and mass spectrometers for structural analysis that performs precision mass analysis such as LCMS-IT-TOF and LCMS-Q-TOF (all manufactured by Shimadzu Corporation).

When a peptide fragment containing an amino acid sequence of a CDR2 region specific to mogamulizumab can be detected, a mogamulizumab can be identified and quantified. Based on a mass spectrometry result, in order to identify mogamulizumab, an existing database can also be used. Further, it is also possible to identify mogamulizumab by identifying an amino acid sequence of a peptide fragment using multistage mass spectrometry or the like.

When identification and quantification of mogamulizumab are performed based on a detection result, the number of amino acid residues of a peptide to be detected is preferably about 5 - 30, and more preferably about 7 - 25.

When concentration of mogamulizumab is quantified, an amount of the mogamulizumab can be calculated based on a peak area or a peak intensity of a detected peptide fragment ion (in the case of multistage MS, a fragment ion obtained by cleavage of a parent ion). For example, based on a correlation between a predetermined calibration curve and a peak area, or a correlation between a peak area derived from an internal standard added to a sample and a peak area derived from the sample, concentration of peptide fragments in the sample is calculated, and, based on the concentration of the peptide fragments, an amount and concentration of mogamulizumab are calculated.

In mass spectrometry, by protease digestion followed by performing, for example, purification using cation exchange resin (WCX and MCX), highly sensitive analysis of a specific peptide of mogamulizumab can be performed.

### [Peptide Containing Amino Acid of CDR2 Region]

A peptide fragment to be detected in the method of the present invention has an amino acid sequence containing an amino acid derived from a CDR2 region of a heavy chain or a light chain of mogamulizumab.

Amino acid sequence information of mogamulizumab are published, and information about amino acid sequences of a heavy chain and a light chain, Fab and Fc domains, CDR regions, and a disulfide bond can be obtained. Further, according to the method of the present invention, contact between mogamulizumab and a protease is limited, and thus, a type of a peptide obtained by selective protease digestion is also limited. Therefore, a person skilled in the art can predict an amino acid sequence of a peptide fragment digested by a specific protease.

Therefore, according to the method of the present invention, detection and quantification of mogamulizumab can also be performed by performing detection of multiple predicted peptide fragments concurrently in parallel. However, for a more convenient measurement and in order to shorten time required for measurement and to reduce cost, it is preferable to select an optimal peptide fragment in mogamulizumab. When an optimal peptide fragment is known, it is possible to determine a mass spectrometry condition suitable only for detection of the peptide fragment and it becomes possible to provide such information.

Therefore, the method of the present invention includes, as one embodiment, selecting a peptide fragment containing an amino acid of a CDR2 region suitable for quantification of mogamulizumab. Further, another embodiment of the present invention provides a method that includes detecting a peptide fragment containing an amino acid of a CDR2 region selected as suitable for quantification of mogamulizumab.

For example, among peptide obtained when mogamulizumab is digested using a trypsin of a mass spectrometry grade, for example, the Trypsin Gold (manufactured by Promega Corporation) using the method of the present invention, peptides predicted to be suitable for mass spectrometry are peptides expressed by SEQ ID NO: 3 - 9. However, in tests performed by the present inventors, concentration-dependent quantitative detection of these peptides could not be performed in serum samples, presumably due to interference of endogenous antibodies.

FSGVPDR (SEQ ID NO: 3)
FTISR (SEQ ID NO: 4)
FSGSGSGTDFTLK (SEQ ID NO: 5)
NSLYLQMNSLR (SEQ ID NO: 6)
HSDGNFAFGYWGQGTLVTVSSASTK (SEQ ID NO: 7)
GLEWVATISSASTYSYYPDSVK (SEQ ID NO: 8)
NIVHINGDTYLEWYLQPKGQSPQLLIYK (SEQ ID NO: 9)

In contrast, among peptides obtained when Trypsin TPCK-Treated (manufactured by Sigma Corporation) was used as a protease, peptides predicted to be suitable for mass spectrometry are peptides expressed by SEQ ID NO: 10 - 14. As a result of actually performing detection of these peptides, a good result was obtained with the peptide SYYPDSVK (SEQ ID NO: 10).

SYYPDSVK (SEQ ID NO: 10)
GMSWVR (SEQ ID NO: 11)
ISRVEAEDVGVY (SEQ ID NO: 12)
YLQKPGQSPQLLIYK (SEQ ID NO: 13)
NIVHINGDTYLEW (SEQ ID NO: 14)

Therefore, when a monoclonal antibody as a measurement target is mogamulizumab, it is preferable to select a peptide having an amino acid sequence expressed in SEQ ID No: 10 as a detection target. In this case, for example, detection can be performed by multiple reaction monitoring using a triple quadrupole mass spectrometer (for example, LCMS-8050 manufactured by Shimadzu Corporation) with conditions described in Table 1 as indicators.

**[Table 1]**

| Peptide sequence | Parent ion m/z | Fragment ion m/z | Q1 pre bias | Q2 CE | Q3 pre bias |
|---|---|---|---|---|---|
| SYYPDSVK | 479.70 | 708.40 | -19 | -16 | -36 |
| SYYPDSVK | 479.70 | 545.30 | -19 | -19 | -40 |
| SYYPDSVK | 479.70 | 251.10 | -19 | -15 | -17 |
| SYYPDSVK | 479.70 | 223.20 | -19 | -22 | -24 |

### [Kit]

The present invention relates to a kit for quantitative detection of mogamulizumab using the above-described high-speed liquid chromatography-mass spectrometry (LC-MS), the kit including: a porous body for immobilizing mogamulizumab as a measurement target; fine particles onto which a protease having trypsin activity and chymotrypsin activity is immobilized; a reaction container for selectively digesting the mogamulizumab by bringing the porous body into contact with the fine particles; and a buffer solution that is introduced into the reaction container together with the fine particles and the porous body and is for causing a digestion reaction by the protease to occur.

Measurement using mass spectrometry enables very high-precision analysis. On the other hand, proper sample preparation and setting of appropriate analysis conditions are very important. For example, in order to more conveniently obtain an accurate examination result at a clinical site, the present invention provides a kit that can be used for implementing the above method.

The porous body and the fine particles included in the kit of the present invention are as described above. The reaction container is not particularly limited as long as the reaction container is a container capable of allowing the mogamulizumab immobilized on the porous body to be in contact with a protease immobilized on the fine particles in a liquid phase. However, considering that the reaction container is for preparing a sample to be detected using mass spectrometry, the reaction container is preferably a microtube or a plate. Considering reaction processes such as mixing using a vortex or a rotator performed for a reaction and filtration for separation of a peptide and the porous body and the fine particles after the reaction, a person skilled in the art can envision an appropriate reaction container.

The buffer solution included in the kit of the present invention is introduced into the reaction container together with the fine particles and the porous body and is for causing a digestion reaction by the protease to occur, and provides a reaction condition suitable for protease digestion. Reaction conditions can be suitably determined depending on, for example, a protease to be selected, and composition of the buffer solution can also be suitably determined.

The kit of the present invention can further include a filtration membrane for removing the porous body and the fine particles after the protease digestion reaction and extracting a product of the digestion reaction together with the buffer solution. This is because it is necessary to remove the porous body and the fine particles in order to subject a target peptide fragment obtained by the protease digestion to mass spectrometry.

The filtration membrane preferably functions as "a bottom of the reaction container" that substantially does not allow the buffer solution and peptides generated by protease digestion to pass through under a condition that a pressure or a centrifugal force is not applied, and functions as "a strainer" that allows the buffer solution and the peptides to pass through during an operation such as centrifugal separation.

A condition of centrifugal separation in order for the filtration membrane to allow the buffer solution and the peptides to pass through is not particularly limited, and, for example, a range of 3,000 - 10,000 g is preferable. As the filtration membrane that can be suitably used in the kit of the present invention, for example, a filtration membrane made of polyvinylidene fluoride (PVDF) (low-binding hydrophilic PVDF, pore diameter: 0.2 µm, manufactured by Millipore Corporation) can be used.

When the kit is a kit for simultaneously processing multiple specimens, for example, a kit can be used in which the filtration membrane is made of PVDF, and a housing material is a polyacrylonitrile resin, for example, Barex (registered trademark) (manufactured by Mitsui Fine Chemicals Inc.).

The kit of the present invention can further include an instruction manual describing use of the kit and/or conditions of mass spectrometry for detecting mogamulizumab.

The kit of the present invention can further include one or more internal standard peptides. The internal standard peptides provide more reliable analysis results by being analyzed at the same time as specimens or separately under the same conditions as specimens. The internal standard peptides contain a specific amino acid sequence of mogamulizumab as a measurement target, and are peptides generated by digestion by the protease included in the kit of the present invention. The internal standard peptides may include those labeled with a stable isotope amino acid. In this case, since mass spectrometry quantification conditions are different as compared with peptides that do not contain isotopes, it is preferable to enclose quantification conditions for internal standards.

For example, when the measurement target is mogamulizumab, the internal standard peptides can include a peptide having an amino acid sequence of SEQ ID NO: 10.

By using the kit of the present invention, preparation operation of a peptide fragment for identification and quantification of mogamulizumab can be more conveniently performed, and automation using devices can also be easily achieved. In particular, when a protease is provided as a component of the kit in a state of being immobilized on surfaces of the fine particles, the preparation operation of the peptide fragment can be further simplified.

### [Recording Medium and Method Package]

The present invention further provides a computer readable recording medium and a method package. The recording medium is for use in the method of the present invention, and data for executing mass spectrometry is recorded in the recording medium, the data including data of a parent ion, a fragment ion, and a voltage applied to electrodes of a mass spectrometer, with respect to a peptide obtained by protease digestion of the mogamulizumab. The method package is for quantitative detection of mogamulizumab using liquid chromatography-mass spectrometry (LC-MS), and includes the recording medium.

In the present specification, the term "method package" means independently distributable package that includes, in a readable form, an analysis condition of liquid chromatography-mass spectrometry with respect to a specific measurement target. By importing the data contained in the method package into LC-MS, it is possible to perform analysis with an optimum measurement condition obtained after detailed examination. The method package can include an instruction manual of the recording medium.

As described above, while measurement using mass spectrometry enables very high-precision analysis, analysis conditions can be completely different depending on target ions. Therefore, although setting appropriate analysis conditions is very important, it is extremely difficult, and enormous time is required for setting the conditions. By preparing these conditions beforehand, convenience of a user who actually performs mass spectrometry can be improved. The present applicant has so far provided method packages for these analyses using LC-MS in order to allow a user to more easily perform mass spectrometry of, for example, agricultural chemicals or animal medicines. Therefore, the present invention also provides a method package for identification and quantification of mogamulizumab in a sample using LC-MS.

That is, the present invention provides a computer readable recording medium. The recording medium is for use in a method for detecting mogamulizumab by analyzing a peptide fragment using liquid chromatography-mass spectrometry (LC-MS), the peptide fragment being obtained by performing selective protease digestion of mogamulizumab by bringing a porous body, in which the mogamulizumab as a measurement target is immobilized in pores, into contact with fine particles, on which a protease having trypsin activity and chymotrypsin activity is immobilized. In the recording medium, data for executing the mass spectrometry is recorded. The data is not limited, but may include, for example, at least data of a parent ion, a fragment ion, and voltages applied to electrodes of a mass spectrometer, for example, triple quadrupoles (a first quadrupole, a second quadrupole, and a third quadrupole), with respect to a peptide that is obtained by protease digestion of the monoclonal antibody and has an amino acid sequence containing an amino acid of a CDR2 region. The recording medium can further include data such as an expected retention time. The expected retention time and the voltage data are numerical values that vary depending on equipment to be used and measurement conditions, and are preferably provided in accordance with the equipment. Further, to facilitate understanding by a person skilled in the art, for a numerical value that varies depending on a condition, it is preferable to also provide a variation range of the numerical value.

The recording medium may be of any form, and is not particularly limited. Examples of the recording medium include disks and memories capable of magnetically or optically recording information.

More specifically, the above method package can include, for example, the following information and software functions.

- Optimized (*) parent ion m/z value
- Optimized fragment ion m/z value
- Optimized Q1 pre bias voltage value
- Optimized Q2 collision energy voltage value
- Optimized Q3 pre bias voltage value
- Expected retention time of a target ion and mass spectrometry time
- Quantitative value conversion formula
- Analysis result report output function
*: Each condition item was actually measured, and one with the highest ionic strength and a most reproducible m/z value is adopted, and this is taken as an optimal value.

The present invention also provides a method package for detection of mogamulizumab using liquid chromatography-mass spectrometry (LC-MS), the method package including the above-described recording medium. The method package can include an instruction manual of the recording medium.

An example of the above-described recording medium or method package is one that includes only information specific to a specific monoclonal antibody. Therefore, when the monoclonal antibody is, for example, mogamulizumab, it is possible to provide a recording medium or a method package in which conditions suitable for analyzing mogamulizumab are described. In this case, the data included in the recording medium can be related to, for example, analysis conditions with respect to a peptide having an amino acid sequence of SEQ ID NO: 10.

The method package may describe data common to multiple mass spectrometers or may describe various data suitable for analysis using a specific mass spectrometer.

The recording medium or method package can be provided together with the above-described kit of the present invention above or separately from the kit.

### [Examples]

In the following, the present invention is further described in detail based on Examples. However, the present invention is not limited by these Examples.

### [Immobilization of Protease]

A protease was immobilized on fine particles by the following steps.

### 1. FG Beads Cleaning

200 mg of FG beads NHS (manufactured by Tamagawa Seiki Co., Ltd.) is subjected to centrifugal separation (15,000 g X 5 minutes, 4 °C), and supernatant isopropanol for preservation is removed. Supernatant, including also floating substances that do not precipitated, is carefully removed.

### 2. Preparation of Enzyme

Trypsin Gold 1 mg (manufactured by Promega Corporation) or Trypsin TPCK 5 mg (manufactured by Sigma-Aldrich Corporation) is opened and dissolved in 25 mM HEPES-NaOH at pH 7.0 cooled to 4 °C. After the dissolution, the solution is put in a 50 ml centrifuge tube, which is then placed on ice. The enzyme is washed once with 25 mM HEPES-NaOH at pH 7.0 and is collected as much as possible. A final buffer volume is about 25 ml.

### 3. FG Beads Cleaning

10 ml of ice-cooled methanol is added, and, in an ice-cooled ultrasonic cleaning machine, a suspension of FG beads is confirmed and then is subjected to centrifugal separation (15,000 g X 5 minutes, 4 °C), and supernatant methanol is removed.

### 4. Enzyme Immobilization Reaction

A Trypsin Gold or Trypsin TPCK solution is added to 200 mg of a precipitate of FG beads. After a suspension of FG beads is confirmed using an ice-cooled ultrasonic washer, vortex is continuously performed for 30 minutes at a minimum speed that allows the suspension to be maintained.

After allowing a reaction to proceed for 30 minutes, centrifugal separation (15,000 g X 5 minutes, 4 °C) is performed, and supernatant is removed. The supernatant is collected and a coupling efficiency confirmation test based on a BCA assay is performed.

### 5. Active Group Block

25 ml of 1M 2-aminoethanol hydrochloride at pH 8.0 is added to 200 mg of a precipitate of FG beads. After a suspension of FG beads is confirmed using an ice-cooled ultrasonic washing machine, vortex is continuously performed at a minimum speed that allows the suspension to be maintained. After blocking by allowing a reaction to proceed for 30 minutes, centrifugal separation is performed and supernatant is removed.

### 6. Enzyme Beads Cleaning

25 ml of 25 mM HEPES-NaOH and 50 mM NaCl at pH 7.0 is added to 200 mg of a precipitate of FG beads. After a suspension of FG beads is confirmed using an ice-cooled ultrasonic washing machine, vortex is continuously performed at a minimum speed that allows the suspension to be maintained. After 5 minutes of washing, centrifugal separation is performed and supernatant is removed.

### 7. Storage

25 mM Tris-HCl at pH 8.0 is added such that a protease final concentration is 0.5 µg/µl. After a suspension of FG beads is confirmed using an ice-cooled ultrasonic washer, the solution is dispensed to 500 µl. Thereafter, the solution is stored at -80 °C.

### [Protease Digestion]

Protease digestion of a monoclonal antibody in a plasma sample was performed by the following steps.

### 1. Collection of Monoclonal Antibody from Plasma

20 µl of a plasma is diluted with 180 µl of PBS + 0.1% n-octyl-β-D-thioglucoside (manufactured by Dojin Chemical).

40 µl of a 50% suspension of Protein G Ultralink resin (manufactured by Pierce Corporation) is added. By slowly rotating with a vortex or a rotary mixer at a room temperature for 1 - 2 hours, antibody molecules in the plasma are bound to the resin. By centrifugal separation, the resin is precipitated and a supernatant is discarded.

By adding 200 µl of PBS + 0.1% n-octyl-β-D-thioglucoside and washing three times, proteins in the plasma nonspecifically bound to the resin are washed. Next, by washing once with 200 µl of PBS, a surfactant is removed.

### 2. Protease Reaction

200 µl of 25 mM Tris-HCl at pH 8.0 is added to a tube in which the Protein G Ultralink resin remains. Next, 80 µl of beads on which the above prepared trypsin (Trypsin Gold or Trypsin TPCK) is immobilized is added to the tube, which is set in a rotator and is slowly rotated at 37 °C for 6 hours.

After reaction, by performing filtration with a 0.2 µm low binding hydrophilic PVDF membrane (manufactured by Millipore Corporation), the Protein G resin and the beads on which the trypsin is immobilized are removed together, and a reaction solution is collected.

### [Mass Spectrometry]

An antibody drug was spiked into a commercially available plasma (manufactured by Sigma Corporation) and a peptide obtained by protease digestion was detected. As a monoclonal antibody, mogamulizumab (drug name: POTELIGEO, Kyowa Hakko Kirin Co., Ltd.) was used. Conditions of mass spectrometry are as follows.

### <HPLC Conditions (Nexera LC30A Liquid Chromatography System)>

Solvent A: 0.1% formic acid; solvent B: 0.1% formic acid + acetonitrile
Flow rate: 0.5 ml/minute
Gradient time: 1% B (1.5 minutes), 1 - 40% B gradient (5 minutes), 95% B (5 minutes), 1% B (5 minutes)
Column: L-column 2 ODS, 2 x 50 mm (Chemical Substance Evaluation and Research Organization)
Column temperature: 50 °C

### <MS Interface Conditions (LCMS-8050 (Shimadzu Corporation))>

Nebulizer gas: 3 L/minute
Heating gas: 10 L/minute
Drying gas: 10 L/minute
Interface temperature: 300 °C
DL temperature: 250 °C
Heat block temperature: 400 °C

### [Detection of Digested Fragment using Trypsin Gold]

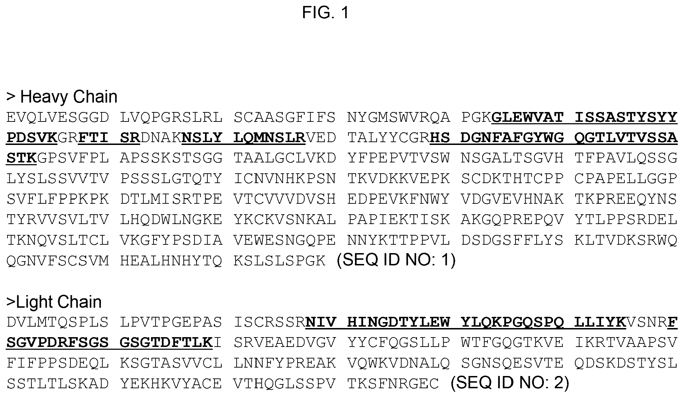

Fig. 1 illustrates amino acid sequences (SEQ ID NO: 1 and 2) of a heavy chain and a light chain of mogamulizumab, and sequences (SEQ ID NO: 3 - 9) of cleavage fragments due to Trypsin Gold used for quantification and positions of the sequences (SEQ ID NO: 3 - 9) in the amino acid sequence of the heavy chain or the light chain.

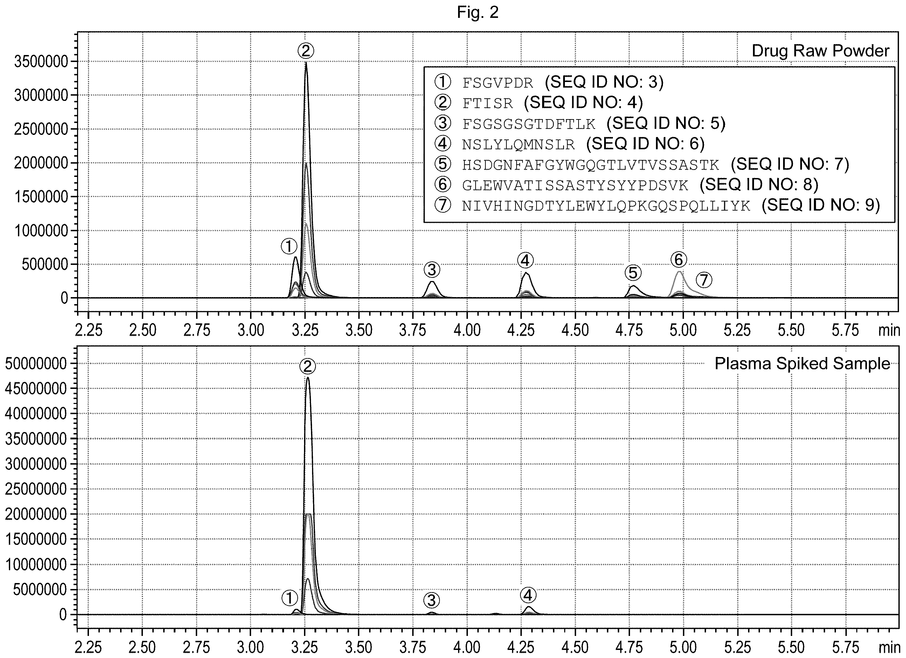

Fig. 2 illustrates results of subjecting the Trypsin Gold cleavage fragments (SEQ ID NO: 3 - 9) of mogamulizumab to MRM chromatogram according to the above-described analysis conditions. When mogamulizumab raw powder was directly analyzed (upper figure), peaks corresponding to addition amounts were observed. However, in a sample obtained by spiking mogamulizumab into a plasma (lower figure), the number of detectable peaks decreased.

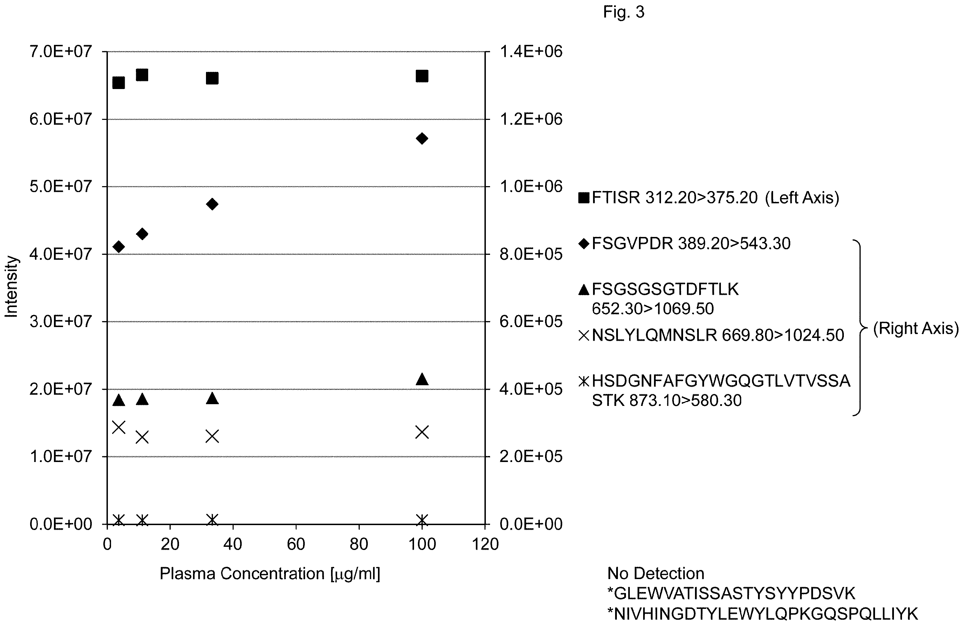

Fig. 3 illustrates results of a preparing calibration curves based on peaks detected with Trypsin Gold cleavage fragments (SEQ ID NO: 3 - 9) spiked into a plasma. As can be seen from Fig. 3, when Trypsin Gold is used as a protease, it was not possible to prepare calibration curves according to an amount of peptide fragments spiked into a plasma, and it was found that none of these peptide fragments is suitable for concentration-dependent quantitative detection.

### [Detection of Digested Fragment using Trypsin TPCK]

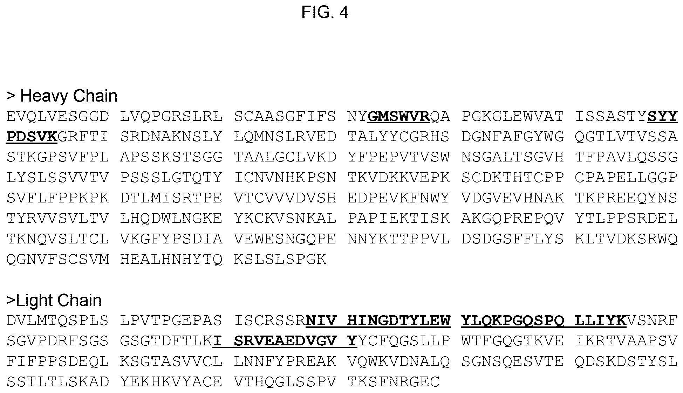

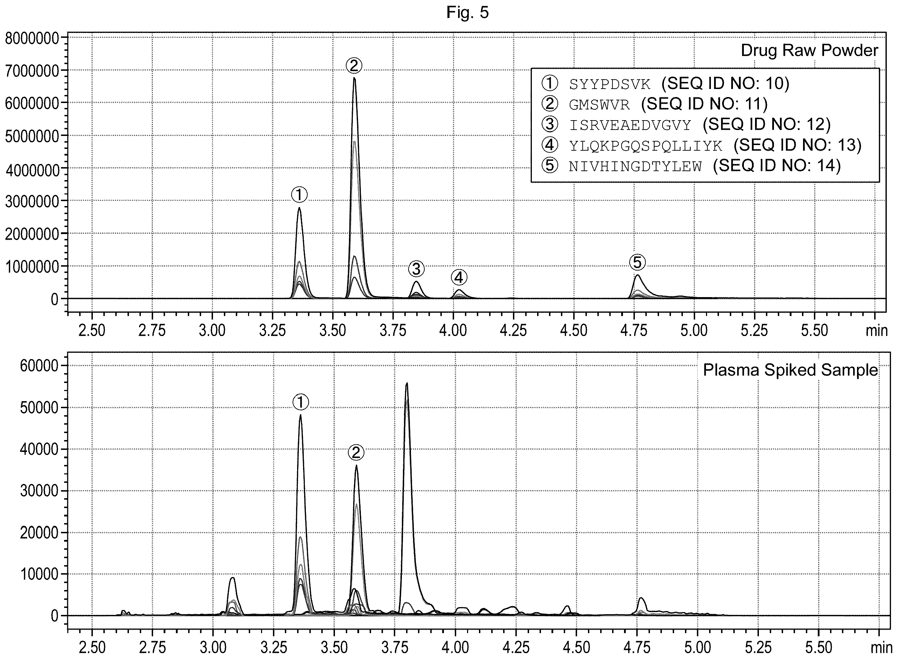

Fig. 4 illustrates sequences (SEQ ID NO: 10 - 14) of Trypsin TPCK cleavage fragments used in quantification of mogamulizumab, and positions of the sequences in the amino acid sequences of the heavy chain and the light chain. Fig. 5 illustrates results of analyzing the Trypsin TPCK cleavage fragments (SEQ ID NO: 10 - 14) of mogamulizumab under the same conditions as above. From the results of Fig. 5, in both the case where mogamulizumab raw powder was directly analyzed (upper figure) and the case where mogamulizumab was spiked into a plasma (lower figure), peaks derived from five kinds of peptide fragments were confirmed.

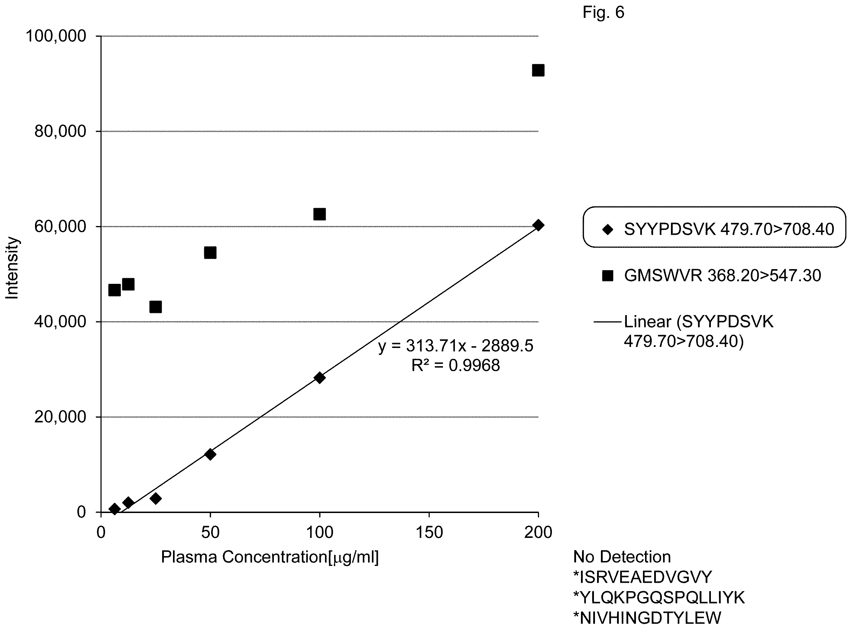

When calibration curves were prepared using Trypsin TPCK cleavage fragments (SEQ ID NO: 10 - 14) spiked into a plasma, as illustrated in Fig. 6, a calibration curve suitable for quantitative detection was obtained with peptide SYYPDSVK (SEQ ID NO: 10), and it was found to be optimal for quantification of mogamulizumab.

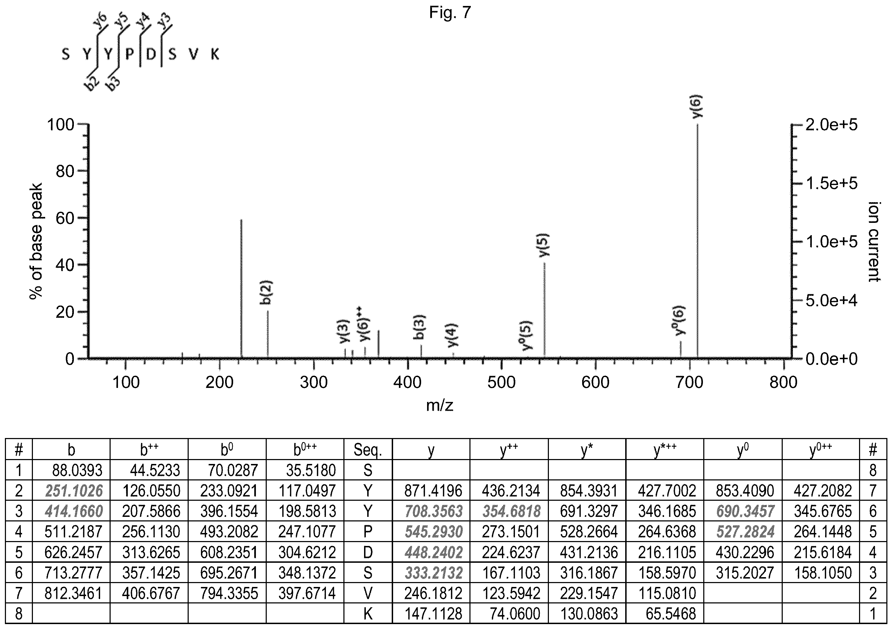

Next, the peptide SYYPDSVK (SEQ ID NO: 10) was selected as a mogamulizumab target peptide in precision mass spectrometry and structure identification by precision mass spectrometry was performed, and the results are shown in Fig. 7. From the results of Fig. 7, it was confirmed that the structure as predicted from the peptide sequence can be identified.

It was confirmed that, as a result of a structural scientific analysis, the detected peptide SYYPDSVK (SEQ ID NO: 10) is derived from an Fv region, and is positioned on an outermost side in a three-dimensional structure of a human immunoglobulin molecule obtained in Protein Data Bank.

### [Industrial Applicability]

According to the method of the present invention, mogamulizumab in a sample can be quantified by selectively digesting the mogamulizumab using a specific protease and subjecting a resulting peptide fragment to mass spectrometry. The method of the present invention allows easy operation and can ensure reproducibility and quantitativeness.

Currently, 40 antibody drugs are on the market, and it is said that there are about 300 antibody drugs are in preclinical trials, more than 2,000 antibody drugs in all clinical trials including animal tests, and more than 5,000 antibody drugs when those in research stage and seeds are included. The present invention can provide an extremely versatile analysis method for antibody drugs, and can contribute to acceleration of future antibody drug development.

### [Sequence List]

PCT_monoclonal_antibody_20150601_145926_1.txt

## Claims

1. A method for quantifying mogamulizumab, the method comprising:
a step of bringing a porous body having pores in which the mogamulizumab is immobilized into contact with a plurality of fine particles onto which a protease is immobilized, such that selective protease digestion of the mogamulizumab occurs; and
a step of detecting a peptide fragment which is obtained from the selective protease digestion and includes an amino acid derived from a CDR2 region of a heavy chain or a light chain of the mogamulizumab,
wherein the protease has trypsin activity and chymotrypsin activity and the fine particles have an average particle size larger than an average pore diameter of the porous body.

2. The method according to claim 1, wherein the protease is a trypsin that has not been subjected to a reductive methylation treatment.

3. The method according to claim 1 or 2, wherein the peptide fragment includes an amino acid sequence of SEQ ID NO: 10.

4. A kit for quantitatively detecting mogamulizumab using liquid chromatography mass spectrometry (LC-MS), the kit comprising:
a porous body for immobilizing the mogamulizumab;
a plurality of fine particles onto which a protease having trypsin activity and chymotrypsin activity is immobilized;
a reaction container for bringing the porous body into contact with the fine particles to selectively digest the mogamulizumab; and
a buffer solution to be introduced into the reaction container with the fine particles and the porous body for causing a digestion reaction of the protease to occur.

5. The kit according to claim 4, wherein the protease is a trypsin that has not been subjected to a reductive methylation treatment.

6. The kit according to claim 4 or 5, further comprising
an internal standard peptide having an amino acid sequence of SEQ ID NO: 10.

7. A non-transitory computer readable medium having stored thereon a program that, when executed by a computer, causes the computer to address devices to execute the method according to any one of claims 1-3, wherein data for executing mass spectrometry is recorded, and the data includes data of a parent ion, a fragment ion, and a voltage applied to electrodes of a mass spectrometer, with respect to a peptide obtained by the selective protease digestion of the mogamulizumab.

8. A method package for quantitatively detecting mogamulizumab, the method package comprising the non-transitory computer readable medium according to claim 7.

## Patentansprüche

1. Verfahren zum Quantifizieren von Mogamulizumab, wobei das Verfahren Folgendes umfasst:
einen Schritt des Inkontaktbringens eines porösen Körpers, der Poren aufweist, in denen das Mogamulizumab immobilisiert ist, mit einer Vielzahl von Feinteilchen, auf denen eine Protease immobilisiert ist, sodass ein selektiver Proteaseverdau des Mogamulizumab eintritt; und
einen Schritt des Detektierens eines Peptidfragments, das durch den selektiven Proteaseverdau erhalten wird und eine Aminosäure umfasst, die von einer CDR2-Region einer Schwerkette oder einer Leichtkette des Mogamulizumab abgeleitet ist,
wobei die Protease Trypsinaktivität und Chymotrypsinaktivität aufweist und die mittlere Teilchengröße der Feinteilchen größer als ein mittlerer Porendurchmesser des porösen Körpers ist.

2. Verfahren nach Anspruch 1, wobei die Protease ein Trypsin ist, das keiner reduktiven Methylierungsbehandlung unterzogen wurde.

3. Verfahren nach Anspruch 1 oder 2, wobei das Peptidfragment eine Aminosäuresequenz der SEQ ID NO: 10 umfasst.

4. Set zum quantitativen Detektieren von Mogamulizumab unter Verwendung von Flüssigchromatographie-Massenspektrometrie (LC-MS), wobei das Set Folgendes umfasst:
einen porösen Körper zum Immobilisieren von Mogamulizumab;
eine Vielzahl von Feinteilchen, auf denen eine Protease mit Trypsinaktivität und Chymotrypsinaktivität immobilisiert ist;
einen Reaktionsbehälter zum Inkontaktbringen des porösen Körpers mit den Feinteilchen, um Mogamulizumab selektiv zu verdauen; und
eine Pufferlösung, die in den Reaktionsbehälter mit den Feinteilchen und dem porösen Körper eingeführt wird, um eine Verdaureaktion der Protease zu bewirken.

5. Set nach Anspruch 4, wobei die Protease ein Trypsin ist, das keiner reduktiven Methylierungsbehandlung unterzogen wurde.

6. Set nach Anspruch 4 oder 5, das außerdem ein inneres Standardpeptid mit einer Aminosäuresequenz der SEQ ID NO: 10 umfasst.

7. Nicht transitorisches computerlesbares Medium, auf dem ein Programm gespeichert ist, das bei der Ausführung durch einen Computer den Computer dazu veranlasst, Vorrichtungen anzusteuern, um ein Verfahren nach einem der Ansprüche 1 bis 3 auszuführen, wobei Daten zum Ausführen von Massenspektrometrie aufgezeichnet werden und die Daten Daten eines Ausgangsions, eines Fragmentions und einer Spannung, die an die Elektroden eines Massenspektrometers angelegt wird, in Bezug auf ein Peptid, das durch den selektiven Proteaseverdau von Mogamulizumab erhalten wurde, umfassen.

8. Verfahrenspaket zum quantitativen Detektieren von Mogamulizumab, wobei das Verfahrenspaket ein nicht transitorisches computerlesbares Medium nach Anspruch 7 umfasst.

## Revendications

1. Procédé pour quantifier le mogamulizumab, le procédé comprenant :
une étape consistant à amener un corps poreux ayant des pores dans lesquels le mogamulizumab est immobilisé en contact avec une pluralité de fines particules sur lesquelles une protéase est immobilisée, de telle sorte qu'une digestion de protéase sélective du mogamulizumab se produit ; et
une étape de détection d'un fragment peptidique résultant de la digestion de protéase sélective et comprenant un acide aminé résultant d'une région CDR2 d'une chaîne lourde ou d'une chaîne légère du mogamulizumab,
dans lequel la protéase a une activité de trypsine et une activité de chymotrypsine et les fines particules ont une taille de particule moyenne supérieure à un diamètre de pore moyen du corps poreux.

2. Procédé selon la revendication 1, dans lequel la protéase est une trypsine qui n'a pas été soumise à un traitement de méthylation réductrice.

3. Procédé selon la revendication 1 ou 2, dans lequel le fragment peptidique comprend une séquence d'acides aminés de SEQ ID NO : 10.

4. Kit de détection quantitative du mogamulizumab en utilisant la spectrométrie de masse par chromatographie en phase liquide (LC-MS), le kit comprenant :
un corps poreux pour immobiliser le mogamulizumab ;
une pluralité de fines particules sur lesquelles une protéase ayant une activité de trypsine et une activité de chymotrypsine est immobilisée ;
un contenant de réaction pour amener le corps poreux en contact avec les fines particules pour digérer sélectivement le mogamulizumab ; et
une solution tampon à introduire dans le contenant de réaction avec les fines particules et le corps poreux pour provoquer la survenance d'une réaction de digestion de la protéase.

5. Kit selon la revendication 4, dans lequel la protéase est une trypsine qui n'a pas été soumise à un traitement de méthylation réductrice.

6. Kit selon la revendication 4 ou 5, comprenant en outre
un peptide standard interne ayant une séquence d'acides aminés de SEQ ID NO : 10.

7. Support lisible par ordinateur non transitoire sur lequel est stocké un programme qui, lorsqu'il est exécuté par un ordinateur, amène l'ordinateur à adresser des dispositifs pour exécuter le procédé selon l'une quelconque des revendications 1 à 3, dans lequel des données pour exécuter la spectrométrie de masse sont enregistrées, et les données comprennent des données d'un ion parent, d'un ion fragment et d'une tension appliquée à des électrodes d'un spectromètre de masse, par rapport à un peptide obtenu par une digestion de protéase sélective du mogamulizumab.

8. Ensemble de procédé pour détecter quantitativement le mogamulizumab, l'ensemble de procédé comprenant le support non transitoire lisible par ordinateur selon la revendication 7.


### SEQUENCE LISTING

<110> Shimadzu Corporation
   National Cancer Center
<120> Method for Quantifying a Monoclonal Antibody
<130> PH-6200-PCT
<160> 14
<170> PatentIn version 3.5
<210> 1
   <211> 449
   <212> PRT
   <213> Artificial
<220>
   <223> antibody heavy chain
<400> 1
   Glu Val Gln Leu Val Glu Ser Gly Gly Asp Leu Val Gln Pro Gly Arg
   1               5                   10                  15
   Ser Leu Arg Leu Ser Cys Ala Ala Ser Gly Phe Ile Phe Ser Asn Tyr
               20                  25                  30
   Gly Met Ser Trp Val Arg Gln Ala Pro Gly Lys Gly Leu Glu Trp Val
           35                  40                  45
   Ala Thr Ile Ser Ser Ala Ser Thr Tyr Ser Tyr Tyr Pro Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Val Glu Asp Thr Ala Leu Tyr Tyr Cys
                   85                  90                  95
   Gly Arg His Ser Asp Gly Asn Phe Ala Phe Gly Tyr Trp Gly Gln Gly
               100                 105                 110
   Thr Leu Val Thr Val Ser Ser Ala Ser Thr Lys Gly Pro Ser Val Phe
           115                 120                 125
   Pro Leu Ala Pro Ser Ser Lys Ser Thr Ser Gly Gly Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Ile Cys Asn Val Asn His Lys Pro
           195                 200                 205
   Ser Asn Thr Lys Val Asp Lys Lys Val Glu Pro Lys Ser Cys Asp Lys
       210                 215                 220
   Thr His Thr Cys Pro Pro Cys Pro Ala Pro Glu Leu Leu Gly Gly Pro
   225                 230                 235                 240
   Ser Val Phe Leu Phe Pro Pro Lys Pro Lys Asp Thr Leu Met Ile Ser
                   245                 250                 255
   Arg Thr Pro Glu Val Thr Cys Val Val Val Asp Val Ser His Glu Asp
               260                 265                 270
   Pro Glu Val Lys Phe Asn Trp Tyr Val Asp Gly Val Glu Val His Asn
           275                 280                 285
   Ala Lys Thr Lys Pro Arg Glu Glu Gln Tyr Asn Ser Thr Tyr Arg Val
       290                 295                 300
   Val Ser Val Leu Thr Val Leu His Gln Asp Trp Leu Asn Gly Lys Glu
   305                 310                 315                 320
   Tyr Lys Cys Lys Val Ser Asn Lys Ala Leu Pro Ala Pro Ile Glu Lys
                   325                 330                 335
   Thr Ile Ser Lys Ala Lys Gly Gln Pro Arg Glu Pro Gln Val Tyr Thr
               340                 345                 350
   Leu Pro Pro Ser Arg Asp Glu Leu Thr Lys Asn Gln Val Ser Leu Thr
           355                 360                 365
   Cys Leu Val Lys Gly Phe Tyr Pro Ser Asp Ile Ala Val Glu Trp Glu
       370                 375                 380
   Ser Asn Gly Gln Pro Glu Asn Asn Tyr Lys Thr Thr Pro Pro Val Leu
   385                 390                 395                 400
   Asp Ser Asp Gly Ser Phe Phe Leu Tyr Ser Lys Leu Thr Val Asp Lys
                   405                 410                 415
   Ser Arg Trp Gln Gln Gly Asn Val Phe Ser Cys Ser Val Met His Glu
   420 425 430
   Ala Leu His Asn His Tyr Thr Gln Lys Ser Leu Ser Leu Ser Pro Gly
   435 440 445
   Lys
<210> 2
   <211> 219
   <212> **PRT**
   <213> Artificial
<220>
   <223> antibody light chain
<400> 2
   Asp Val Leu Met Thr Gln Ser Pro Leu Ser Leu Pro Val Thr Pro Gly
   1 5 10 15
   Glu Pro Ala Ser Ile Ser Cys Arg Ser Ser Arg Asn Ile Val His Ile
   20 25 30
   Asn Gly Asp Thr Tyr Leu Glu Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Phe Gln Gly
   85 90 95
   Ser Leu Leu Pro Trp Thr Phe Gly Gln Gly Thr Lys Val Glu Ile Lys
   100 105 110
   Arg Thr Val Ala Ala Pro Ser Val Phe Ile Phe Pro Pro Ser Asp Glu
   115 120 125
   Gln Leu Lys Ser Gly Thr Ala Ser Val Val Cys Leu Leu Asn Asn Phe
   130 135 140
   Tyr Pro Arg Glu Ala Lys Val Gln Trp Lys Val Asp Asn Ala Leu Gln
   145 150 155 160
   Ser Gly Asn Ser Gln Glu Ser Val Thr Glu Gln Asp Ser Lys Asp Ser
   165 170 175
   Thr Tyr Ser Leu Ser Ser Thr Leu Thr Leu Ser Lys Ala Asp Tyr Glu
   180 185 190
   Lys His Lys Val Tyr Ala Cys Glu Val Thr His Gln Gly Leu Ser Ser
   195 200 205
   Pro Val Thr Lys Ser Phe Asn Arg Gly Glu Cys
   210 215
<210> 3
   <211> 7
   <212> PRT
   <213> Artificial
<220>
   <223> antibody fragment
<400> 3
   Phe Ser Gly Val Pro Asp Arg
   1 5
<210> 4
   <211> 5
   <212> PRT
   <213> Artificial
<220>
   <223> antibody fragment
<400> 4
   Phe Thr Ile Ser Arg
   1 5
<210> 5
   <211> 13
   <212> PRT
   <213> Artificial
<220>
   <223> antibody fragment
<400> 5
   Phe Ser Gly Ser Gly Ser Gly Thr Asp Phe Thr Leu Lys
   1 5 10
<210> 6
   <211> 11
   <212> PRT
   <213> Artificial
<220>
   <223> antibody fragment
<400> 6
   Asn Ser Leu Tyr Leu Gln Met Asn Ser Leu Arg
   1               5                   10
<210> 7
   <211> 25
   <212> PRT
   <213> Artificial
<220>
   <223> antibody fragment
<400> 7
   His Ser Asp Gly Asn Phe Ala Phe Gly Tyr Trp Gly Gln Gly Thr Leu
   1               5                   10                  15
   Val Thr Val Ser Ser Ala Ser Thr Lys
               20                  25
<210> 8
   <211> 22
   <212> PRT
   <213> Artificial
<220>
   <223> antibody fragment
<400> 8
   Gly Leu Glu Trp Val Ala Thr Ile Ser Ser Ala Ser Thr Tyr Ser Tyr
   1               5                   10                  15
   Tyr Pro Asp Ser Val Lys
               20
<210> 9
   <211> 28
   <212> PRT
   <213> Artificial
<220>
   <223> antibody fragment
<400> 9
   Asn Ile Val His Ile Asn Gly Asp Thr Tyr Leu Glu Trp Tyr Leu Gln
   1               5                   10                  15
   Pro Lys Gly Gln Ser Pro Gln Leu Leu Ile Tyr Lys
               20                  25
<210> 10
   <211> 8
   <212> PRT
   <213> Artificial
<220>
   <223> antibody fragment
<400> 10
   Ser Tyr Tyr Pro Asp Ser Val Lys
   1               5
<210> 11
   <211> 6
   <212> PRT
   <213> Artificial
<220>
   <223> antibody fragment
<400> 11
   Gly Met Ser Trp Val Arg
   1               5
<210> 12
   <211> 12
   <212> PRT
   <213> Artificial
<220>
   <223> antibody fragment
<400> 12
   Ile Ser Arg Val Glu Ala Glu Asp Val Gly Val Tyr
   1               5                   10
<210> 13
   <211> 15
   <212> PRT
   <213> Artificial
<220>
   <223> antibody fragment
<400> 13
   Tyr Leu Gln Lys Pro Gly Gln Ser Pro Gln Leu Leu Ile Tyr Lys
   1               5                   10                  15
<210> 14
   <211> 13
   <212> PRT
   <213> Artificial
<220>
   <223> antibody fragment
<400> 14
   Asn Ile Val His Ile Asn Gly Asp Thr Tyr Leu Glu Trp
   1               5                   10